# EUROPEAN PATENT APPLICATION

(11) **EP 3 351 297 A1**
(43) Date of publication of application: **25.07.2018**
(21) Application number: 16846602.7
(22) Date of filing: 16.09.2016
(51) Int. Cl.: B01D 53/52, B01D 53/14, B01D 53/72, C12P 1/04

(54) **GAS TREATMENT METHOD AND APPARATUS**

(30) Priority: 17.09.2015 JP 2015183977
(71) Applicant: Sekisui Chemical Co., Ltd., Osaka-shi, Osaka 530-8565 (JP)
(72) Inventor: FUJIMORI, Yoji, Tsukuba-shi Ibaraki 300-4292 (JP); ISHII, Tetsuya, Tsukuba-shi Ibaraki 300-4292 (JP)
(74) Representative: Ter Meer Steinmeister & Partner
(86) International application number: PCT/JP2016/077375
(87) International publication number: WO 2017/047731

(57) **Abstract**

It is an object of the present invention to downsize facilities for removing hydrogen sulfide and oxygen in gas and reduce facility cost. Syngas g containing hydrogen sulfide and oxygen as target components for removal or reduction in concentration is contacted with a first material containing transition metal 41 and subsequently with a second material containing transition metal 42 in a first mode. The syngas g is contacted with the second material containing transition metal 42 and subsequently with the first material containing transition metal 41 in a second mode. The first mode and the second mode are alternately executed. In the first mode, iron oxide of the first material containing transition metal 41 reacts with the hydrogen sulfide to become iron sulfide that is reactable with the oxygen and iron sulfide of the second material containing transition metal 42 reacts with the oxygen to become iron oxide that is reactable with the hydrogen sulfide. In the second mode, the iron oxide of the second material containing transition metal 42 reacts with the hydrogen sulfide to become iron sulfide and the iron sulfide of the first material containing transition metal 41 reacts with the oxygen to become iron oxide.

## Description

### FIELD OF THE INVENTION

The present invention relates to a method and apparatus for treating gas containing hydrogen sulfide and oxygen, and particularly relates to a method and apparatus for treating gas to remove or reduce concentration of the hydrogen sulfide and the oxygen in the gas.

### BACKGROUND OF THE INVENTION

For example, in Patent Document 1, valuable materials such as ethanol are produced by fermentative action of anaerobic microorganisms using syngas (synthetic gas) containing carbon monoxide and hydrogen. The syngas contains components such as hydrogen sulfide and oxygen. These components may be harmful to the microorganisms, and therefore, it is mentioned to remove these components in a pretreatment step.

### PRIOR ART DOCUMENTS

### PATENT DOCUMENTS

Patent Document 1: Japanese Patent Application Publication No. 2014-050406 (Paragraph 0102)

### SUMMARY OF THE INVENTION

### PROBLEM TO BE SOLVED BY THE INVENTION

Usually, a dedicated desulfurization device is used for removing hydrogen sulfide and a dedicated deoxidization device is used for removing oxygen. It is costly to be equipped with these two devices. When a copper catalyst is used as the deoxidization device, it is required to heat it at a high temperature.

In view of the above, it is an object of the present invention to remove or reduce concentration of the hydrogen sulfide and the oxygen in the gas by a simple structure, thereby downsizing the facility and reducing the cost.

### MEANS FOR SOLVING THE PROBLEMS

To solve the problems mentioned above, the present invention provides a method for treating gas containing hydrogen sulfide and oxygen as target components for removal or reduction in concentration, characterized in that the method comprises steps of: alternately executing a first mode and a second mode; in the first mode, the gas being contacted with a first material containing transition metal and subsequently contacted with a second material containing transition metal, the first material containing transition metal reacting with a first gas component to become reactable with a second gas component, the second material containing transition metal reacting with the second gas component to become reactable with the first gas component, the gas components being the hydrogen sulfide and the oxygen in the gas; and in the second mode, the gas being contacted with the second material containing transition metal and subsequently contacted with the first material containing transition metal, the second material containing transition metal reacting with the first gas component to become reactable with the second gas component, the first material containing transition metal reacting with the second gas component to become reactable with the first gas component.

Transition metals constituting the first and second materials containing transition metals may be iron (Fe) or manganese (Mn), for example.

Components of the first and second materials containing transition metals may be transition metal oxide such as iron oxide and manganese oxide or transition metal sulfide such as iron sulfide and manganese sulfide.

For example, iron oxide reacts with hydrogen sulfide to become iron sulfide (Formulas 1 and 2).

Fe₂O₃•3H₂O+3H₂S→Fe₂S₃+6H₂O (Formula 1)

FeO+H₂S→-FeS+H₂O (Formula 2)

The produced iron sulfide is reactable with oxygen (O₂). By this reaction, the iron sulfide returns to the iron oxide (Formulas 3 to 5).

Fe₂S₃+3/2O₂+nH₂O-Fe₂O₃•nH₂O+3S →-Fe₂O₃+nH₂O+3S (Formula 3)

4FeS+7O₂→2Fe₂O₃+4SO₂ (Formula 4)

2FeS+3O₂→2FeO+2SO₂ (Formula 5)

In a case where the reactant is iron sulfide, iron oxide is produced by reaction with oxygen (Formulas 3 to 5). The produced iron oxide returns to the iron sulfide by reaction with the hydrogen sulfide (Formulas 1 and 2).

Accordingly, in the first mode, in a case where a main component of the first material containing transition metal is iron oxide (first transition metal oxide) and a main component of the second material containing transition metal is iron sulfide (second transition metal sulfide) at the start of the first mode, for example, the hydrogen sulfide is removed or reduced in concentration by the iron oxide in the first material containing transition metal and the iron oxide (first transition metal oxide) is converted into the iron sulfide (first transition metal sulfide). Subsequently, the oxygen is removed or reduced in concentration by the iron sulfide (second transition metal sulfide) in the second material containing transition metal and the iron sulfide (second transition metal sulfide) is converted into the iron oxide (second transition metal oxide). In the second mode, the hydrogen sulfide is removed or reduced in concentration by the iron oxide (second transition metal oxide) in the second material containing transition metal and the iron oxide (second transition metal oxide) is converted into the iron sulfide (second transition metal sulfide). Subsequently, the oxygen is removed or reduced in concentration by the iron sulfide (first transition metal sulfide) in the first material containing transition metal and the iron sulfide (first transition metal sulfide) is converted into the iron oxide (first transition metal oxide). By switching between and executing the first mode and the second mode alternately, the hydrogen sulfide and the oxygen in the target gas can be removed or reduced in concentration in a continuous manner. Thereby, a dedicated deoxidization device (removing device dedicated to the second gas component) such as copper catalyst may not be required or used less frequently or downsized. Therefore, facility cost can be reduced.

Manganese oxide reacts with hydrogen sulfide to become manganese oxide. The manganese sulfide reacts with oxygen to become manganese oxide. Accordingly, in a case where the transition metal of the first and second materials containing transition metal is manganese (Mn), as with iron, the hydrogen sulfide and the oxygen in the target gas can be removed or reduced in concentration in a continuous manner by switching between and executing the first mode and the second mode alternately.

Preferably, a molar content rate of the first gas component is higher than a molar content rate of the second gas component in the gas before the treatment.

Thereby, in both first and second modes, of the hydrogen sulfide and the oxygen in the gas, a highly-contained gas component contained in higher molar content rate can be treated to be removed first, and subsequently, a low-contained gas component contained in lower molar content rate can be treated to be removed.

Preferably, the first gas component (highly-contained gas component) is hydrogen sulfide.

Preferably, the second gas component (low-contained gas component) is oxygen.

Preferably, a hydrogen sulfide content and an oxygen content in the gas before the contact are measured and switching between the first mode and the second mode is performed based on results of the measurements.

From the hydrogen sulfide content and the oxygen content, quantity ratio such as molar fraction of desulfurizing component such as iron oxide and deoxidizing component such as iron sulfide in the first and second materials containing transition metals can be calculated or estimated. Thereby, timing for switching modes can be determined.

Preferably, a start-up mode is performed before the switching between the first mode and the second mode, the gas being contacted with the second material containing transition metal without being contacted with the first material containing transition metal beforehand, to make the second material containing transition metal reactable with the second gas component by reacting with the first gas component.

Thereby, in an initial state (before starting the start-up mode) both of the first and second materials containing transition metals can be a component that is reactable with the first gas component (iron oxide, for example), and the second material containing transition metal can be converted into a component that is reactable with the second gas component (iron sulfide, for example) in the start-up mode. And then, the first mode is executed. Separately in the start-up mode, the second gas component in the gas is removed or reduced in concentration by a dedicated device for removing the second gas component. To put it another way, the dedicated device for removing the second gas component is required mainly in the start-up mode only, and therefore, the device can be downsized or can be used less frequently.

The reaction in which the iron sulfide reacts with the oxygen and returns to the iron oxide is an exothermal reaction. Such reaction may be hard to occur when the quantity of oxygen is extremely small or when the temperature is low. To facilitate the smooth start-up of this reaction, a reacting part may be heated. Alternatively, oxygen gas may be temporarily blown into the reacting part to increase oxygen concentration, thereby inducing the reaction, and thereby generating heat. Thus, the reaction can proceed in a smooth manner.

Preferably, the gas after the treatment is provided to liquid culture medium for culturing gas-utilizing microorganisms therein. The gas-utilizing microorganisms intake CO or the like in the gas and produce valuable materials by fermentation. The gas-utilizing microorganisms can be cultured in a stable manner by supplying the target gas to the liquid culture medium after removing or reducing concentration of the oxygen or the like.

The hydrogen sulfide contains sulfur (S) that is an element necessary for the gas-utilizing microorganisms, and essentially there is no need to remove the hydrogen sulfide. However, when treating the gas to remove oxygen or acethylene using a noble metal catalyst or a base metal catalyst, sulfur (S) can be a typical poisoning substance to these catalysts. Therefore, it is necessary to reduce the hydrogen sulfide to a ppm level or to a ppb level depending on the catalyst. The gas such as the syngas commonly contains hydrogen sulfide in a concentration of higher than few tens of ppm. Therefore, if the concentration of the hydrogen sulfide is to be reduced to few ppm to ppb level, the cost therefor will be very high. Moreover, it is required to supplement the sulfur (S) needed by the gas-utilizing microorganisms by adding sodium sulfide or the like in a separate step. This is an inefficient system to supplement the sulfur (S) once removed.

On the other hand, according to the method of the present invention except for the start-up mode, it is not necessarily required to use a catalyst for removing oxygen or the like. Therefore, there will be no problem even if some hydrogen sulfide remains in the gas. This will lighten the burden of removal facility. At the same time, adding may not be required or an amount to be added can be reduced in facilities for adding sulfur compound. Thereby, synergistic effects can be expected.

The present invention provides an apparatus for treating gas containing hydrogen sulfide and oxygen as target components for removal or reduction in concentration, the apparatus including: a first desulfurizing and deoxidizing part having a first material containing transition metal therein; a second desulfurizing and deoxidizing part having a second material containing transition metal therein; a mode switching part alternately switching between a first mode and a second mode; in the first mode, the gas being passed through the first desulfurizing and deoxidizing part and subsequently through the second desulfurizing and deoxidizing part, the first material containing transition metal reacting with a first gas component to become reactable with a second gas component, the second material containing transition metal reacting with the second gas component to become reactable with the first gas component, the gas components being the hydrogen sulfide and the oxygen in the gas; and in the second mode, the gas being passed through the second desulfurizing and deoxidizing part and subsequently through the first desulfurizing and deoxidizing part, the second material containing transition metal reacting with the first gas component to become reactable with the second gas component, the first material containing transition metal reacting with the second gas component to become reactable with the first gas component.

### ADVANTAGEOUS EFFECTS OF THE INVENTION

According to the present invention, an apparatus dedicated for removing the second gas component of the hydrogen sulfide and oxygen in the target gas is not required, a frequency of use of the apparatus can be reduced or the apparatus can be downsized. Therefore, facilities can be downscaled and facility cost can be reduced.

### BRIEF DESCRIPTION OF THE DRAWINGS

FIG. 1 is a block diagram of a valuable materials producing system according to a first embodiment of the present invention, showing the system in a start-up mode.
FIG. 2 is a block diagram of the valuable materials producing system in a first mode.
FIG. 3 is a block diagram of the valuable materials producing system in a second mode.

### MODE FOR CARRYING OUT THE INVENTION

One embodiment of the present invention will be described hereinafter with reference to the drawings.

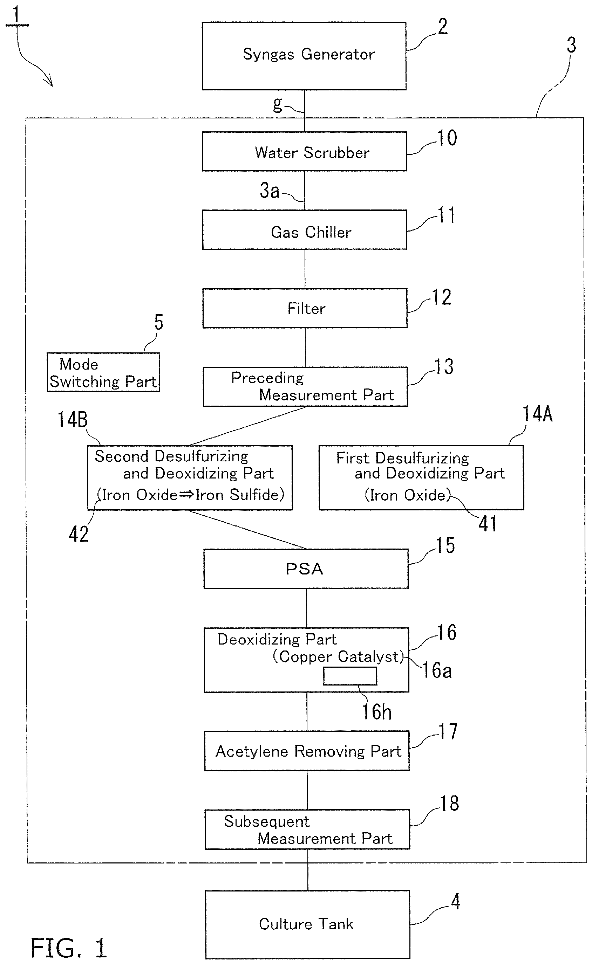

FIGS. 1 to 3 show a valuable materials producing system 1 according to one embodiment of the present invention. As shown in FIG. 1, the valuable materials producing system 1 includes a gas treatment part 3 and a culture tank 4. A syngas generator 2 is provided before the valuable materials producing system 1. The syngas generator 2 is a waste disposal facility in this embodiment. Wastes may include municipal wastes, tires, biomass, wooden chips and plastic wastes. The syngas generator 2 is provided with a melting furnace. In the melting furnace, the wastes are burnt by a highly-concentrated oxygen gas and decomposed to a low-molecular level. Eventually, syngas g (target gas) is generated.

The syngas g derived from wastes includes CO and H₂ as useful components. Moreover, the syngas g includes hydrogen sulfide (H₂S) and oxygen (O₂) as target substances in target components for removal or reduction in concentration. The syngas g further includes CO₂, water content (H₂O), solid impure substance, naphthalene, benzene (BTEX) and acetylene (C₂H₂) or the like as target substances in target components for removal or reduction in concentration.

The syngas g derived from wastes is generally hydrogen sulfide rich. That is, a molar content rate of hydrogen sulfide is higher than a molar content rate of oxygen in the syngas g. In this embodiment, the hydrogen sulfide in the syngas g constitutes a "first gas component" or a "higher-contained gas component" and the oxygen in the syngas g constitutes a "second gas component" or a "lower-contained gas component".

The gas treatment part 3 includes a passage 3a for the syngas g. The gas passage 3a is provided with a water scrubber 10, a gas chiller 11, a filter 12, a preceding measurement part 13, an acetylene removing part 17 and a subsequent measurement part 18 in this order from an upstream side. Desulfurizing and deoxidizing parts 14A, 14B, a PSA (pressure-swing adsorption) 15 and a deoxidizing part 16 are provided between the preceding measurement part 13 and the acetylene removing part 17.

Although not shown in detail in the drawings, the preceding measurement part 13 includes a concentration measuring portion and an integral processing portion. A concentration of the hydrogen sulfide and a concentration of the oxygen in the syngas g are measured in the concentration measuring portion. Measured values of hydrogen sulfide concentration and measured values of oxygen concentration are respectively integrated over a certain measurement time in the integral processing portion. The time-integrated values respectively correspond to hydrogen sulfide content and oxygen content of the syngas g that passed through the preceding measurement part 13 over the measurement time.

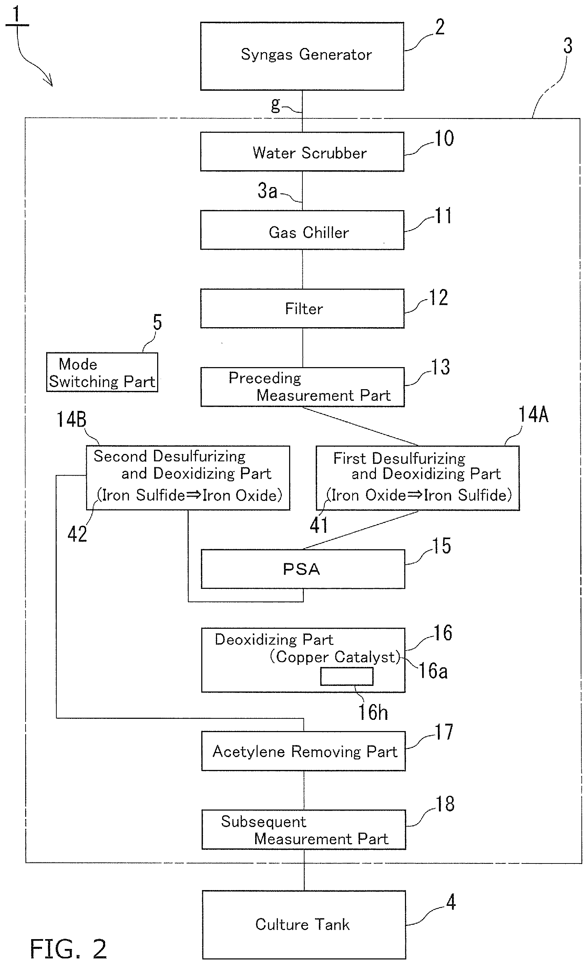

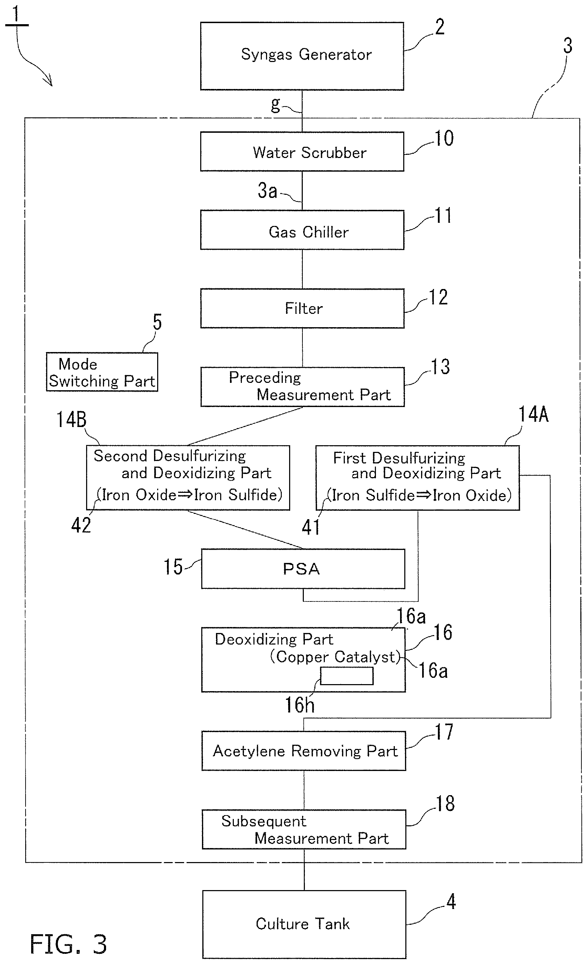

The first desulfurizing and deoxidizing part 14A is provided with a first material containing transition metal 41. The first material containing transition metal 41 reacts with hydrogen sulfide to become a product that is reactable with oxygen. And by reacting with the oxygen, the product returns to a reactant that is reactable with hydrogen sulfide. Specifically, as shown in FIGS. 1 and 2, the first material containing transition metal 41 in an initial state (before a beginning of a first mode to be described later) is composed of iron oxide. As shown in FIG. 2, in the first mode, preferably a majority of the first material containing transition metal 41 is converted into iron sulfide. As shown in FIG. 3, preferably a majority of the first material containing transition metal 41 at a beginning of a second mode to be described later is composed of iron sulfide. In the second mode, preferably a majority of the iron sulfide is returned to the iron oxide.

The second desulfurizing and deoxidizing part 14B is provided with a second material containing transition metal 42. The second material containing transition metal 42 reacts with oxygen to become a product that is reactable with hydrogen sulfide. And by reacting with the hydrogen sulfide, the product returns to a reactant that is reactable with oxygen. Specifically, as shown in FIG. 1, the second material containing transition metal 42 in an initial state (before a beginning of a start-up mode to be described later) is composed of iron oxide as with the first material containing transition metal 41. In the start-up mode, preferably a majority of the second material containing transition metal 42 is converted into iron sulfide. As shown in FIG. 2, preferably a majority of the second material containing transition metal 42 at the beginning of the first mode is composed of iron sulfide. In the first mode, preferably a majority of the iron sulfide is returned to the iron oxide. As shown in FIG. 3, preferably a majority of the second material containing transition metal 42 at the beginning of the second mode is composed of iron oxide. In the second mode, preferably a majority of the iron oxide is returned to the iron oxide.

PSA 15 is provided with zeolite, silica gel, activated carbon or the like as adsorbent.

The deoxidizing part 16 is provided with a deoxidizing agent 16a. A copper catalyst, for example, is used as the deoxidizing agent 16a. The deoxidizing part 16 is provided with a heater 16h. Heating temperature of the heater 16h may be set at around 150 to 400 degrees C, for example.

In place of the copper, platinum (Pt), nickel (Ni) or the like may be used as the deoxidizing agent 16a.

The acetylene removing part 17 is provided with a noble metal such as palladium (Pd), platinum (Pt) or the like as an acetylene removal catalyst.

The subsequent measurement part 18 includes a concentration measuring portion and an integral processing portion in a similar manner to the preceding measurement part 13.

The gas treatment part 3 is further provided with a mode switching part 5. As shown in FIGS. 1 to 3, the mode switching part 5 switches between three modes of the gas passage 3a. Connection order from the preceding measurement part 13 to the acetylene removing part 17 differs according to the mode. As shown in FIG. 1, in the start-up mode, a connection is made from the preceding measurement part 13, to the second desulfurizing and deoxidizing part 14B, to the PSA 15, to the deoxidizing part 16, and to the acetylene removing part 17 in this order from the upstream side. As shown in FIG. 2, in the first mode, a connection is made from the preceding measurement part 13, to the first desulfurizing and deoxidizing part 14A, to the PSA 15, to the second desulfurizing and deoxidizing part 14B, and to the acetylene removing part 17 in this order from the upstream side. As shown in FIG. 3, in the second mode, a connection is made from the preceding measurement part 13, to the second desulfurizing and deoxidizing part 14B, to the PSA 15, to the first desulfurizing and deoxidizing part 14A, and to the acetylene removing part 17 in this order from the upstream side.

The culture tank 4 is connected subsequent to the gas treatment part 3. Liquid culture medium is stored in the culture tank 4. Anaerobic gas-utilizing microorganisms are cultured in the liquid culture medium. Anaerobic bacteria such as those disclosed in the Patent Document 1 given above, International Publication No. WO2011/087380, United States Patent Application Publication No. 2013/0065282 or the like may be used as the gas-utilizing microorganisms. Valuable materials such as ethanol (C₂H₅OH) are produced from the syngas g by metabolism of the gas-utilizing microorganisms.

Though not shown in the drawings, a refiner including a distillation tower is provided subsequent to the culture tank 4.

A method for producing ethanol (valuable material) using the valuable materials producing system 1 will be described hereinafter.

### <Start-up Mode>

As shown in FIG. 1, at a start of an operation of the valuable materials producing system 1, the gas treatment part 3 is set to the start-up mode. That is, prior to switching between the first and second modes, the start-up mode is executed.

A processing before the preceding measurement part 13, a processing at the PSA 15 and a processing after the acetylene removing part 17 in the start-up mode are common to those in the first mode and the second mode. Details of the common processing are to be described in the description of the first mode.

In the start-up mode, the syngas g after the measurement at the preceding measurement part 13 is introduced to the second desulfurizing and deoxidizing part 14B and contacted with the second material containing transition metal 42 without passing through the first desulfurizing and deoxidizing part 14A (eventually without passing through the first material containing transition metal 41). Thereby, the iron oxide constituting the second material containing transition metal 42 is converted into iron sulfide by reaction with the hydrogen sulfide in the syngas g (Formulas 1 and 2).

Fe₂O₃•3H_{2O}+3H₂S→Fe₂S₃+6H₂O (Formula 1)

FeO+H₂S→-FeS+H₂O (Formula 2)

Iron sulfide is reactable with oxygen (Formulas 3 to 5 to be described later). In short, the second material containing transition metal 42 becomes reactable with oxygen (second gas component) by the reaction with the hydrogen sulfide (first gas component). Moreover, by the reactions of Formulas 1 and 2, the hydrogen sulfide in the syngas g can be removed (or reduced in concentration).

Subsequently, after the processing at the PSA 15, the syngas g is introduced to the deoxidizing part 16 to be contacted with the copper catalyst 16a. Thereby, the oxygen in the syngas g is removed (or reduced in concentration). At this time, the deoxidizing agent 16a is heated to about 150 to 400 degrees C, for example, by the heater 16h. Thereby, the removal of the oxygen can be facilitated.

### <Switching from the Start-up Mode to the First Mode>

Measured values of hydrogen sulfide concentration and measured values of oxygen concentration in the syngas g after the start of the start-up mode are respectively integrated over time by the preceding measurement part 13. Thereby, integrated quantities of the hydrogen sulfide and the oxygen in the syngas g that passed through the preceding measurement part 13 before the beginning of the start-up mode can be obtained. From the integrated quantities, a quantity of the iron oxide converted into the iron sulfide in the second material containing transition metal 42 can be calculated or estimated. When preferably a majority (not less than 50 mol %, preferably not less than 90 mol %) of the second material containing transition metal 42 is converted into the iron sulfide, switching to the first mode is performed by the mode switching part 5.

### <First Mode>

The first mode includes steps given below.

As shown in FIG. 2, the syngas g is generated by burning wastes in the syngas generator 2 (Gas Generating Step). The syngas g is introduced to the gas treatment part 3.

In the gas treatment part 3, the syngas g is purified by removing or reducing concentration of the target substance in the syngas g.

Specifically, water soluble impure substances in the syngas g are removed in the water scrubber 10 first.

Next, in the gas chiller 11, the water content (H₂O) and naphthalene or the like in the syngas g are removed. The water content may be left in a certain quantity for a deoxidizing step (Formula 3) or the like to be described later.

Next, solid impure substances in the syngas g are removed by the filter 12.

Next, a concentration of the hydrogen sulfide and a concentration of the oxygen in the syngas g are measured in the preceding measurement part 13 (Measuring Step).

Subsequently, the syngas g is introduced to the first desulfurizing and deoxidizing part 14A and contacted with the first material containing transition metal 41 (First Contacting Step). Thereby, reactions as described in Formulas 1 and 2 occur between the iron oxide constituting the first material containing transition metal 41 and the hydrogen sulfide in the syngas g to remove (or reduce concentration of) the hydrogen sulfide in the syngas g. Moreover, the iron oxide constituting the first material containing transition metal 41 is converted into the iron sulfide.

Fe₂O₃•3H_{2O}+3H₂S→Fe₂S₃+6H₂O (Formula 1)

FeO+H₂S→FeS+H₂O (Formula 2)

The iron sulfide is reactable with the oxygen (Formulas 3 to 5 to be described later). In other words, the first material containing transition metal 41 reacts with the hydrogen sulfide (first gas component) in the syngas g to become reactable with the oxygen (second gas component).

Next, the benzene (BTEX) and the CO₂ or the like in the syngas g are removed by adsorption in the PSA 15.

Subsequently, the syngas g is introduced to the second desulfurizing and deoxidizing part 14B and contacted with the second material containing transition metal 42 (Second Contacting Step). Thereby, reactions as described in Formulas 3 to 5 occur between the iron sulfide in the second material containing transition metal 42 and the oxygen in the syngas g to remove (or reduce concentration of) the oxygen in the syngas g. And the iron sulfide in the second material containing transition metal 42 is converted into the iron oxide.

Fe₂S₃+3/2O₂+nH₂O-Fe₂O₃•nH₂O+3S →-Fe₂O₃+nH₂O+3S (Formula 3)

4FeS+7O₂→2Fe₂O₃+4SO₂ (Formula 4)

2FeS+3O₂→2FeO+2SO₂ (Formula 5)

The iron oxide is reactable with the hydrogen sulfide (Formulas 1 and 2). In other words, the second material containing transition metal 42 reacts with the oxygen (second gas component) in the syngas g to become reactable with the hydrogen sulfide (first gas component).

Such reaction may be hard to occur when the quantity of oxygen is extremely small or when the temperature remains low. To facilitate the smooth start-up of this reaction, the desulfurizing and deoxidizing part 14B may be heated. Alternatively, concentration of oxygen in oxygen gas blown into the desulfurizing and deoxidizing part 14B may be temporarily increased to induce the reaction, thereby generating heat. Thereby, the reaction can proceed in a smooth manner.

Preferably, a heater or a steamer or the like may be used as a heat source. It is not required to bring the temperature to high. The temperature of about 180 degrees C may be enough. Temporary heating at the starting up is enough because once the oxidation reactions (Formulas 3 to 5) start, the desulfurizing and deoxidizing part 14B is heated by exothermal reaction. Since it is not constant heating required for a catalyst, the running cost reduction effect can be sufficiently achieved.

Subsequently, the syngas g is sent out to the acetylene removing part 17 without passing through the deoxidizing part 16. Since the oxygen can be removed in the second desulfurizing and deoxidizing part 14B, it is not required to use a dedicated deoxidizing part 16.

Acetylene in the syngas g is removed in the acetylene removing part 17.

Subsequently, composition of the syngas g is measured in the subsequent measurement part 18. Particularly, remaining amount of the hydrogen sulfide and the oxygen in the syngas g are measured.

When the hydrogen sulfide or oxygen or the like remain, it is preferable to perform a removal treatment with a hydrogen sulfide remover (PSA) or an oxygen remover (copper catalyst) or the like in a separate step. Since the remaining amount should be small even in this case, a load on the separate step for the removal treatment should be light, and the device configuration can be simplified.

After that, the syngas g is supplied to the liquid culture medium in the culture tank 4. Thereby, the gas-utilizing microorganisms in the culture medium intake CO and H₂ or the like in the syngas g and produce the valuable materials such as ethanol by fermentation (Step of Producing Valuable Materials).

By removing impure substances such as oxygen in the syngas g beforehand, the gas-utilizing microorganisms can be cultured in a stable manner.

A portion of the liquid culture medium in the culture tank 4 is introduced to the distillation tower (not shown) and distilled (Refining Step). Thereby, valuable materials such as ethanol can be extracted.

### <Switching from the First Mode to the Second Mode>

In the preceding measurement part 13, measured values of hydrogen sulfide concentration and measured values of oxygen concentration in the syngas g after the start of the first mode are respectively integrated over time. Thereby, integrated quantities of the hydrogen sulfide and the oxygen in the syngas g that passed through the preceding measurement part 13 after the beginning of the first mode can be obtained. From the integrated quantities, a quantity of the iron oxide converted into the iron sulfide in the first material containing transition metal 41 and a quantity of the iron sulfide converted into the iron oxide in the second material containing transition metal 42 can be calculated or estimated. When preferably a majority (not less than 50 mol %, preferably not less than 90 mol %) of the first material containing transition metal 41 is converted into the iron sulfide, or when preferably a majority (not less than 50 mol %, preferably not less than 90 mol %) of the second material containing transition metal 42 is converted into the iron oxide, switching to the second mode is performed by the mode switching part 5.

### <Second Mode>

As shown in FIG. 3, processing up to the preceding measurement part 13 in the second mode is common to that of the first mode.

In the second mode, the syngas g after the measurement in the preceding measurement part 13 is firstly introduced to the second desulfurizing and deoxidizing part 14B to be contacted with the second material containing transition metal 42 (Second Contacting Step). Thereby, reactions as described in Formulas 1 and 2 occur between the iron oxide in the second material containing transition metal 42 and the hydrogen sulfide in the syngas g to remove (or reduce concentration of) the hydrogen sulfide in the syngas g. And the iron oxide in the second material containing transition metal 42 is converted into the iron sulfide.

Fe₂O₃•3H_{2O}+3H₂S→Fe₂S₃+6H₂O (Formula 1)

FeO+H₂S→-FeS+H₂O (Formula 2)

The iron sulfide is reactable with the oxygen (Formulas 3 to 5). In other words, the second material containing transition metal 42 reacts with the hydrogen sulfide (first gas component) in the syngas g to become reactable with the oxygen (second gas component).

Next, the benzene (BTEX) and the CO₂ or the like are removed by adsorption in the PSA 15.

Subsequently, the syngas g is introduced to the first desulfurizing and deoxidizing part 14A and contacted with the first material containing transition metal 41 (First Contacting Step). Thereby, reactions as described in Formulas 3 to 5 occur between the iron sulfide in the first material containing transition metal 41 and the oxygen in the syngas g to remove (or reduce concentration of) the oxygen in the syngas g. And the iron sulfide in the first material containing transition metal 41 is converted into the iron oxide.

Fe₂S₃+3/2O₂+nH₂O-Fe₂O₃•nH₂O+3S →-Fe₂O₃+nH₂O+3S (Formula 3)

4FeS+7O₂→2Fe₂O₃+4SO₂ (Formula 4)

2FeS+3O₂→2FeO+2SO₂ (Formula 5)

The iron oxide is reactable with the hydrogen sulfide (Formulas 1 and 2). In other words, the first material containing transition metal 41 reacts with the oxygen (second gas component) in the syngas g to become reactable with the hydrogen sulfide (first gas component).

As with the first mode, the desulfurizing and deoxidizing part 14A may be temporarily heated or oxygen gas may be temporarily blown into the desulfurizing and deoxidizing part 14A at the beginning of the reaction.

Subsequently, the syngas g is sent out to the acetylene removing part 17 without passing through the deoxidizing part 16. Since the oxygen can be removed in the first desulfurizing and deoxidizing part 14A, it is not required to use a dedicated deoxidizing part 16.

Processing after the acetylene removing part 17 in the second mode is same as that of the first mode.

### <Switching from the Second Mode to the First Mode>

In the preceding measurement part 13, measured values of hydrogen sulfide concentration and measured values of oxygen concentration in the syngas g after the start of the second mode are respectively integrated over time. Thereby, integrated quantities of the hydrogen sulfide and the oxygen in the syngas g that passed through the preceding measurement part 13 after the beginning of the second mode can be obtained. From the integrated quantities, a quantity of the iron oxide converted into the iron sulfide in the second material containing transition metal 42 and a quantity of the iron sulfide converted into the iron oxide in the first material containing transition metal 41 can be calculated or estimated. When preferably a majority (not less than 50 mol %, preferably not less than 90 mol %) of the second material containing transition metal 42 is converted into the iron sulfide, or when preferably a majority (not less than 50 mol %, preferably not less than 90 mol %) of the first material containing transition metal 41 is converted into the iron oxide, switching to the first mode is performed by the mode switching part 5.

After that, the first mode and the second mode are alternately executed in this manner.

In the valuable materials producing system 1, it is not required to use a dedicated deoxidizing part 16 (lower-contained gas component removing device) except in the start-up mode. Accordingly, use frequency of the deoxidizing part 16 can be constrained, and a required amount of the deoxidizing agent 16a can be reduced. Moreover, a load on the heater 16h can be reduced. Since the deoxidization by the iron sulfide does not require heat, it is not required to provide the desulfurizing and deoxidizing parts 14A, 14B with a heater. Therefore, facility cost can be reduced.

Moreover, since the desulfurizing agent and the deoxidizing agent composed of the materials containing transition metal 41, 42 can be reproduced while being consumed, lives of the desulfurizing agent and the deoxidizing agent can be prolonged.

By using iron oxide as the materials containing transition metal 41, 42 in the initial state, material cost can be reduced and handling can be easy.

The present invention is not limited to the embodiments described above. Various modifications can be made without departing from the scope and spirit of the invention.

For example, the start-up mode may be omitted by using iron sulfide as the second material containing transition metal 42 in the initial state.

The transition metal in the materials containing transition metal 41, 42 is not limited to iron (Fe), but may be manganese (Mn).

At the start of the first mode, the first material containing transition metal 41 may be composed mostly of manganese oxide and the second material containing transition metal 42 may be composed mostly of manganese sulfide.

At the start of the first mode, the first material containing transition metal 41 may be composed mostly of transition metal sulfide such as iron sulfide and manganese sulfide and the second material containing transition metal 42 may be composed mostly of transition metal oxide such as iron oxide and manganese oxide.

The transition metal of the first material containing transition metal 41 and the transition metal of the second material containing transition metal 42 may be different from each other.

The oxygen content of the syngas g may be greater than the hydrogen sulfide content of the syngas g.

The target valuable material to be produced in the culture tank 4 is not limited to ethanol. Alternatively, the target valuable material may be acetic acid or methanol or the like.

The syngas g may be by-product gas of a steel plant (gas from a converter, a blast furnace or the like).

The syngas generator 2 is not limited to the waste disposal facility. Alternatively, the syngas generator 2 may be a steel plant, a coal power plant or the like.

### INDUSTRIAL APPLICABILITY

The present invention may be applied to an ethanol producing system, for example, in which ethanol is produced from syngas generated in an incineration disposal of industrial wastes.

### EXPLANATION OF REFERENCE NUMERALS

- 1: valuable materials producing system
- 2: syngas generator
- 3: gas treatment part
- 3a: gas passage
- 4: culture tank
- 5: mode switching part
- 10: water scrubber
- 11: gas chiller
- 12: filter
- 13: preceding measurement part
- 14A: first desulfurizing and deoxidizing part
- 14B: second desulfurizing and deoxidizing part
- 41: first material containing transition metal
- 42: second material containing transition metal
- 15: PSA
- 16: deoxidizing part
- 16a: deoxidizing agent
- 16h: heater
- 17: acetylene removing part
- 18: subsequent measurement part
- g: syngas (target gas)

## Claims

1. A method for treating gas containing hydrogen sulfide and oxygen as target components for removal or reduction in concentration, **characterized in that** the method comprises steps of:
alternately executing a first mode and a second mode;
in the first mode, the gas being contacted with a first material containing transition metal and subsequently contacted with a second material containing transition metal, the first material containing transition metal reacting with a first gas component to become reactable with a second gas component, the second material containing transition metal reacting with the second gas component to become reactable with the first gas component, the gas components being the hydrogen sulfide and the oxygen in the gas; and
in the second mode, the gas being contacted with the second material containing transition metal and subsequently contacted with the first material containing transition metal, the second material containing transition metal reacting with the first gas component to become reactable with the second gas component, the first material containing transition metal reacting with the second gas component to become reactable with the first gas component.

2. The method for treating gas according to claim 1, wherein a molar content rate of the first gas component is higher than a molar content rate of the second gas component in the gas before the treatment.

3. The method for treating gas according to one of claims 1 and 2, wherein a hydrogen sulfide content and an oxygen content in the gas before the contact are measured and switching between the first mode and the second mode is performed based on results of the measurements.

4. The method for treating gas according to any one of claims 1 to 3, wherein a start-up mode is performed before the switching between the first mode and the second mode, the gas being contacted with the second material containing transition metal without being contacted with the first material containing transition metal beforehand, to make the second material containing transition metal reactable with the second gas component by reacting with the first gas component.

5. The method for treating gas according to any one of claims 1 to 4, wherein the transition metal constituting the first material containing transition metal or the second material containing transition metal comprises iron or manganese.

6. The method for treating gas according to any one of claims 1 to 5, wherein the gas after the treatment is provided to liquid culture medium for culturing gas-utilizing microorganisms therein.

7. An apparatus for treating gas containing hydrogen sulfide and oxygen as target components for removal or reduction in concentration, **characterized in that** the apparatus comprises:
a first desulfurizing and deoxidizing part having a first material containing transition metal therein;
a second desulfurizing and deoxidizing part having a second material containing transition metal therein;
a mode switching part alternately switching between a first mode and a second mode;
in the first mode, the gas being passed through the first desulfurizing and deoxidizing part and subsequently through the second desulfurizing and deoxidizing part, the first material containing transition metal reacting with a first gas component to become reactable with a second gas component, the second material containing transition metal reacting with the second gas component to become reactable with the first gas component, the gas components being the hydrogen sulfide and the oxygen in the gas; and
in the second mode, the gas being passed through the second desulfurizing and deoxidizing part and subsequently through the first desulfurizing and deoxidizing part, the second material containing transition metal reacting with the first gas component to become reactable with the second gas component, the first material containing transition metal reacting with the second gas component to become reactable with the first gas component.
